# EUROPEAN PATENT SPECIFICATION

(11) **EP 2 432 340 B1**
(45) Date of publication and mention of the grant of the patent: **05.04.2017**
(21) Application number: 10777239.4
(22) Date of filing: 21.05.2010
(51) Int. Cl.: A41D 13/00, A61F 13/08

(54) **COMPRESSION GARMENTS AND METHOD OF MANUFACTURE**
KOMPRESSIONSWÄSCHE UND HERSTELLUNGSVERFAHREN
VÊTEMENTS DE COMPRESSION ET LEUR PROCÉDÉ DE FABRICATION

(30) Priority: 21.05.2009 AU 2009902341
(43) Date of publication of application: 28.03.2012
(73) Proprietor: Skins International Trading AG, 6312 Steinhausen (CH)
(72) Inventor: MCLAREN, Jason, Wolli Creek NSW 2205 (AU); HERROD, Amanda, Kensington NSW 2033 (AU); BARTON, Payne, La Jolla CA 92037 (US)
(74) Representative: Caldwell, Judith Margaret
(86) International application number: PCT/AU2010/000611
(87) International publication number: WO 2010/132950

(56) References cited:
- WO-A1-2006/032096
- WO-A1-2006/032096
- WO-A1-2007/112494
- US-A- 5 105 478
- US-A1- 2004 016 041
- US-A1- 2004 016 041
- US-A1- 2004 016 043
- US-A1- 2006 169 004
- US-A1- 2006 272 071
- US-A1- 2008 189 824
- US-A1- 2010 299 799

## Description

### Technical Field

The present invention relates to compression garments and to methods of manufacture. In particular, this invention is concerned with compression garments including upper body garments and lower body garments.

### Background

Prior art compression garments such as those described in US 2010/0299799, WO 2008/142334 and US 2006/0169004 are generally designed to fit the body snugly, but without consideration as to the extent to which muscles increase in bulk and mass during activity. US 2010/0299799 provides a garment having a plurality of panels having different elasticity for producing different compression effects to create a pressure gradient that decreases towards the heart..US 2006/0169004 provides tights designed for supporting muscle masses and for holding knee joints using localized compression effect. Such prior art garments can become non-static or counter-gradient in this situation. Once a person wearing a static compression garment increases muscle mass with activity, the garment can become tighter in the vicinity of the muscle, which can increase as much as 3-5% in volume. This alters the effect of the static compression and can create undesirable effects, in being undesirably tight or in providing more compression in the wrong places. In turn, this can impede circulation and reduce the effect of lymphatic drainage.

Prior art compression garments do not take into consideration difference in the degree of shortening which takes place during activity and exposure to increased eccentric load (lengthening) for some muscle groups.

There is also a need to provide compression garments which, at least in preferred embodiments, can take into consideration the effect of excess pressure in particular areas, in order to alleviate or avoid injury problems.

As one example, one of the most common running injuries is illiotibial band friction syndrome (ITBFS), sometimes known as "runner's knee." The illiotibial band is a thickening of the lateral or outer soft tissue that envelops the leg, known as the fascia. The illiotibial band extends from the hip to the outside of the knee. The muscles that insert into the upper portion of the band are the tensor fascia latae

and a portion of the gluteus maximus and gluteus medius muscles. The injury normally presents as pain and inflammation on the outside of the knee.

The illiotibial band may become tight for various reasons and the pain may be experienced down the side of the leg. Friction may be caused in the femoral area where the illiotibial band crosses the outside of the knee joint.

It has been found that excess pressure provided by a compression garment may cause friction of the illiotibial band over the hip and knee joint, resulting in or adding to ITBFS. This type of injury can occur with repetitive hip and knee flexion during jumping and running sports, in particular.

It is an aim of the present invention, at least in some embodiments, to improve on the compression garments of the prior art by providing compression garments which can reduce the likelihood of injury from ITBFS and/or which can provide focused support and compression on key areas and muscle groups, including the illiotibial band, the gluteus muscles.

### Disclosure of the Invention

The present invention provides a compression garment for clothing a lower body part, the compression garment having a first panel of stretchable material joined to a second panel of stretchable material by a seam, wherein the stretchable material of the second panel has a higher stretch and recovery characteristic compared to the stretchable material of the first panel, and wherein, in use, the first panel of stretchable material is adapted to support a larger, more powerful muscle group than the second panel according to claim 1.

The body part may be a leg, the lower torso, or a combination of these. For example, the compression garment of the invention may comprise shorts or long tights, either as a single garment or in a combination of garments intended to be worn as a suit.

It is disclosed that the compression garment may be made from two or more different elastomeric materials.

It is disclosed that the material of which the compression garment is made may be chosen from a wide variety of fabric or different fabrics. However, the garment is made of panels of fabrics of elastane or similar stretch material, often combined with nylon or polyester or similar stretch materials of 40 to 60 or up to 500 denier material. The fabric is of specific stretch and recovery. The stretch along the warp of the fabric is between 120% and 225% and its number for recovery is between 5% and 40%.

According to an aspect of the invention, the stretchable material of the second panel has a higher stretch and recovery characteristic compared to the stretchable material of the first panel. The characteristic of the second panel maybe achieved in any desirable way. For example, the second panel may be made of material which is of lower denier, or greater elasticity compared to the material of the first panel.

It is disclosed that the first panel of the compression garment can effect a compression value of between 5mm Hg and 25mm Hg. It is also disclosed that the compression garment may be used for therapy and in that case, compression levels may be greater, for example, up to 40mm Hg. In most embodiments of the compression garment, compression will be of a lower grade, being less than 25mm Hg, ranging down to 5mm Hg, for active wear and 30mm Hg, ranging down to 8mm Hg, for inactive or non sports usage.

It is disclosed that the material of the first and/or second panels preferably has a "wicking" effect, so that in use it draws moisture from the body. Such materials are known.

The invention is based on the concept of providing panels in the compression garment to represent patterns of arrangement of muscle structure. This concept takes into account that the larger, more powerful muscle groups have the most muscle fibres, but their degree of shortening is small. Examples of these muscle groups are the gluteals (gluteus maximus), the quads (quadriceps, located at the front of the thighs), the hams (hamstrings, located at the back of the thighs), the calves (located at the back of the lower leg}, the traps (trapezius, located in the upper back of the torso), the lats (latissimus dorsi, mid side back of the torso) and the rectus abdominis (the front abdominals). In preferred embodiments, the first panel fully covers the muscle belly of such muscle groups. It is believed that in this way vasoconstriction of the muscle belly may be reduced and more effective compression over muscle groups with more blood supply may be effected.

It is disclosed that the seam is preferably a flat stitched seam joining panels of elastomeric material. However, the seam is not limited to this. For example, the seam may be a line or ridge of greater thickness than the surrounding area of the compression garment. Thus the seam may be formed by gluing, stitching or any other means.

Stitching is preferably flat stitching using a four or six needle process.

It will be appreciated by one skilled in the art that the compression garments of the invention, in appropriate aspects, can utilise a combination of compressive and high stretch/recovery fabrics in the postero-lateral panels of the waist and thigh. This can help to create a complementary functional mechanism of the oblique muscles, the gluteus maximus, the illiotibial band and the tensor fascial latae.

In preferred embodiments, in relation to the compression garment being a lower body garment, it is preferred that the waist band is made from a panel of stretchable material having chosen stretch and recovery characteristics designed so that the waist band does not need to include an elastic insert in the rear of the garment. In this embodiment, it is optional to have an elastic insert in the front of the garment. It will be appreciated that this is different from prior art arrangements, in which an elastic insert encircles the waist or, where an elastic insert is included in only part of the waist band, the elastic insert is used at the back of the garment rather than at the front. Construction of the type described in relation to the lower body garment of the invention can provide vertical stability.

The embodiment of waistband described above represents an aspect of the invention, but

Optionally, the compression garment of the invention may include one or more mesh inserts, for the purpose in aiding ventilation or to assist in dissipating body heat. In the case of a lower body garment, for example, a mesh insert may be provided located below the waist band at the upper back. It is a further option that there may be a second mesh insert in the crotch area.

### Brief Description of the Drawings

To assist with understanding the present invention, reference will now be made to certain non-limiting embodiments in the accompanying drawings, in which:
Figure 1 shows a front view of a first embodiment of a lower body compression garment in accordance with the invention;
Figure 2 shows a side view of the embodiment of Figure 1;
Figure 3 shows a rear view of the embodiment in Figures 1 and 2;
Figure 4 shows a front view of a first embodiment of an upper body compression garment, not forming part of the claimed invention;
Figure 5 is a side view of the embodiment in Figure 4; and
Figure 6 is a rear view of the embodiment in Figures 4 and 5.

### Detailed Description of the Invention

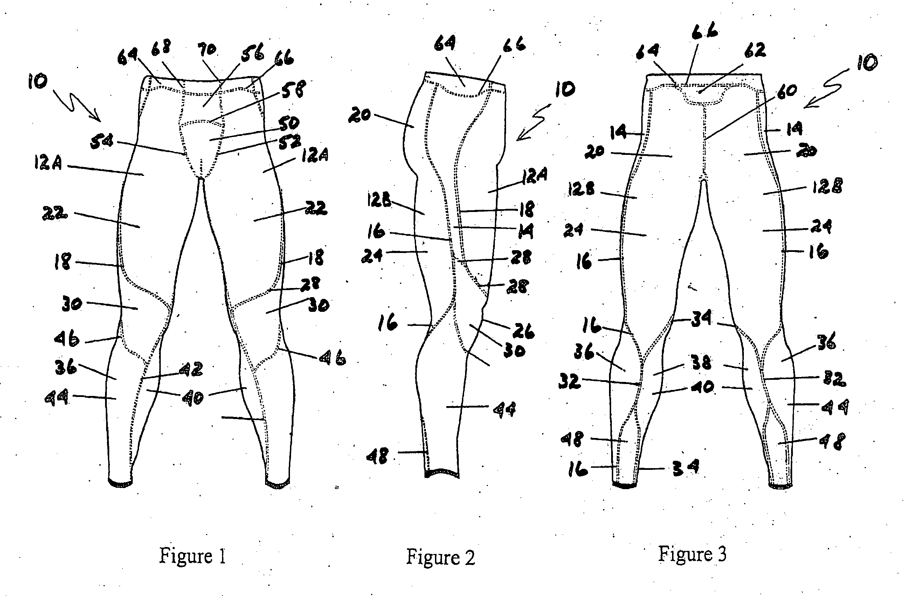

Referring first to Figures 1 to 3, compression garment 10 is shown as a lower body garment in the form of long tights, for clothing a body part being the lower torso, from approximately the wearer's waistline to the wearer's ankles.

Long tights 10 have first panels of stretchable material 12A and 12B and a second panel of stretchable material 14. First panel 12A is joined to second panel 14 by second seam 18 First panel 12B is joined to second panel 14 by first seam 16. Effectively, first panel 12 is divided into two parts, 12A and 12B, panel 12A covering the front of the wearer's thigh as shown in Figure 1 and panel 12B covering the back of the wearer's thigh as shown in Figure 3, with a seam (not shown) joining the panels 12A and 12B running along the inside thigh of the wearer.)

First panels 12A and 12B require more force to stretch and therefore are less elastic than second panel 14.

First panel 12B is adapted to support the gluteus maximus of the wearer, located at 20, and the hamstrings, located at 24. First panel 12A is adapted to support the quadriceps, located at 22

It will be noted from Figures 1 and 2 that second seam 18 is located so that it crosses the upper leg of the wearer above the knee region 26 and so does not impede knee flexion.

It will also be noted from Figure 2 that second panel 14 is located along a substantial part of the illiotibial band of the wearer, terminating above knee region 26 at third seam 28.

In this embodiment, knee region 26 is covered by a further panel 30. This may be a material of similar elasticity to second panel 14, but preferably is made from a material of even greater elasticity than second panel 14, so as not impede knee flexion.

At the lower leg of compression garment 10, seam 16 is continued to wrap around to the rear of the lower leg so that it at least partially divides the triceps surae of the lower leg at 32. Seam 34 similarly travels from the region of the wearer's inner thigh to meet seam 16 at 32. It will be noted that seams 16 and 34 where they merge at 32 effectively divide the calf muscles 36 and 38 of the wearer's lower leg.

In this embodiment, panel 40 is bordered by seam 34 and seam 42 (refer Figure 1) which lies along the bony prominence of the tibia.

Panel 44 is bordered by seam 42, the extension of seam 16 and lower seam 46 to knee panel 30. It is preferred that panels 40 and 44 are made of the same material as first panels 12A and 12B.

As shown in Figure 3, a further panel 48 supports the wearer's achilles tendons, being formed between extensions of seams 16 and 34. Preferably, panel 48 is made from a material of greater elasticity, such as that of second panel 14 or that of knee panel 30.

As shown in Figure 1, long tights 10 have crotch panel 50 joined by seams 52 and 54 two first panels 12A. Crotch panel 50 may be of the same elasticity as first panels 12A or of a different elasticity.

Crotch panel 50 is joined to an upper panel 56 by seam 58. Once again, panel 56 may be of the same elasticity or of a different elasticity compared to the surrounding panels.

As can be seen from Figure 3, seam 60 joins the panels 12B and travels between the gluteus maximus locations 20. Panel 62 lies above seam 60 and below waistband 64. If desired, panel 62 may consist of or may include a mesh panel to assist in ventilation. A further mesh panel may be included in crotch panel 50 or elsewhere located near the wearer's crotch.

As may be seen from each of the Figures 1 to 3, waistband 64 is shaped by seam 66 so that compression garment 10 sits flatly at the wearer's waist.

In this embodiment, waistband 64 has no separate elastic insert, except within the front of waistband 64 between seams 68 and 70.

Compression garment 10 is manufactured by providing each of the respective panels and joining them by each of the respective seams to provide the desired shape for the intended wearer.

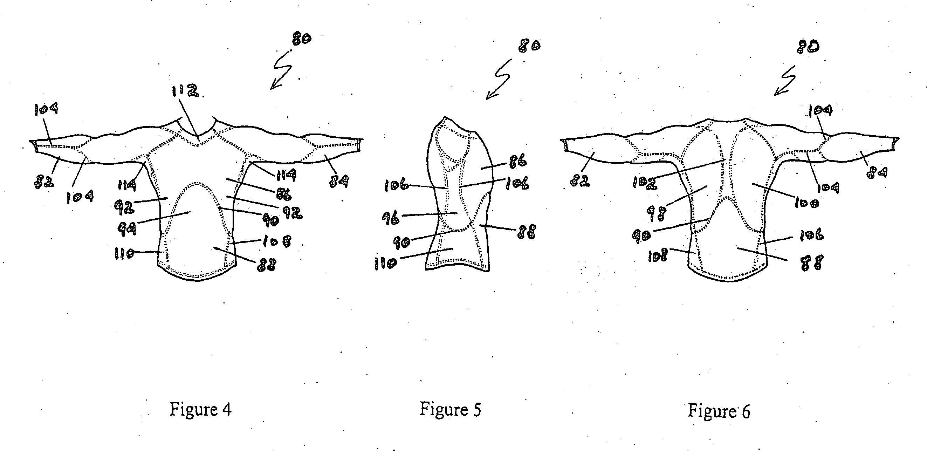

Although Figures 1 to 3 show compression garment 10 in a form of long tights, it will be appreciated that the first and second aspects of the invention may be embodied in a short tight version which is truncated above knee region 26, but this does not form part of the claimed invention.
Turning now to Figures 4 to 6, compression garment 80 is shown in the form of an upper body garment which has long sleeves 82 and 84 which does not form part of the claimed invention. It will be appreciated, however, that long sleeves 82 and 84 are not included or are present in the form of shorter sleeves.

Compression garment 80 has first panel 86 joined to second panel 88 by seam 90. Seam 90 is intended to follow the fibre direction of the external oblique muscles of the wearer, located at 92 in order to assist with twisting movements of the wearer during activity. First panel 86 is of a material of lower elasticity then second panel 88. This provides compression to the upper part of the wearer's torso while allowing greater elasticity over the diaphragm area 94.

As can be seen from Figure 5, seam 90 is intended to travel along the lower extremity of the latissimus dorsi at 96.

As can be seen from Figure 6, seam 90 rises up again at the rear of upper body garment 80 to support the scapular muscles.

In the illustrated non-claimed embodiment, and as can be seen from Figure 6, upper body garment 80 has panels 98 and 100 separated by further panel 102. Panels 98 and 100 are made of the same, relatively low elasticity, material as panel 86. However, panel 102 is made from a higher elasticity material, either the same as that of panel 88 or of a different elasticity, the purpose of panel 102 being to permit proper movement of wearer's back during activity, whilst still providing compression through panels 98 and 100.

In the non-claimed embodiment shown, further seams 104 are provided to help anchor arm muscles.

Side seams 106 are provided to assist in shaping upper body garment 80. Optionally, side seams 106 may permit side panels 108 and 110 to be of different elasticity compared to other panels, such as panel 86 and panels 98 and 100.

As with long tights 10, upper body garment 80 may have mesh inserts to assist in ventilation. These may be used, for example, in neck panel 112 or in underarm panels 114.

All seams referred to in the above embodiments are made using 4-needle flatlock stitching and may serve to anchor muscle groups and assist in compression.

It will be appreciated by those skilled in the art that many modifications and variation may be made to the embodiments described herein without departing from the scope of the invention.

## Claims

1. A compression garment (10) for clothing a lower body part of a wearer, the compression garment having:
a first panel (12) of stretchable material joined to a second panel (14) of stretchable material by a first seam (16) and a second seam (18);
a third panel (30) joined to the second panel (14) by a third seam (28);
the stretchable material of the second panel (14) having a higher stretch and recovery characteristic compared to the stretchable material of the first panel (12);
**characterised in that**, in use, the first panel (12) of stretchable material is adapted to support a larger, more powerful muscle group than the second panel (14), the first panel being divided into two parts, a first part (12b) adapted to support the gluteus maximus and hamstring of the wearer, and a second part (12a) adapted to support the quadriceps of the wearer, the second panel (14) being adapted in use to be located along a substantial part of the illiotibial band of the wearer, and the third panel (28) designed in use to be located over the knee.

2. The compression garment (10) of claim 1, wherein the third panel (30) is of greater elasticity than the second panel (14) so as to allow knee flexion.

3. The compression garment (10) of claim 1 or 2, further including a waist band (64) made from a panel of stretchable material having chosen stretch and recovery characteristics designed so that the waist band does not need to include an elastic insert in the rear of the garment.

4. The compression garment (10) of claim 3, further including an elastic insert in the front of the garment.

5. The compression garment (10) of claim 3 or 4, further including one or more mesh inserts, for the purpose in aiding ventilation or to assist in dissipating body heat.

6. The compression garment (10) of claim 5, wherein the mesh insert (62) is located below the waist band (64) at the upper back.

7. The compression garment (10) of claim 5 or 6, wherein a second mesh insert is provided in the crotch area.

## Patentansprüche

1. Kompressionskleidungsstück (10) zum Bekleiden eines unteren Körperteils eines Trägers, wobei das Kompressionskleidungsstück Folgendes aufweist:
ein erstes Flächengebilde (12) aus dehnbarem Material, das durch eine erste Naht (16) und eine zweite Naht (18) mit einem zweiten Flächengebilde (14) aus dehnbarem Material verbunden ist;
ein drittes Flächengebilde (30), das durch eine dritte Naht (28) mit dem zweiten Flächengebilde (14) verbunden ist;
wobei das dehnbare Material des zweiten Flächengebildes (14), verglichen mit dem dehnbaren Material des ersten Flächengebildes (12), ein höheres Dehn- und Rückstellvermögen aufweist;
**dadurch gekennzeichnet, dass** bei Gebrauch das erste Flächengebilde (12) aus dehnbarem Material dazu ausgelegt ist, eine größere, kräftigere Muskelgruppe zu stützen als das zweite Flächengebilde (14), wobei das erste Flächengebilde in zwei Teile unterteilt ist, einen ersten Teil (12b) der dazu ausgelegt ist, den M. gluteus maximus und die ischiocrurale Muskulatur des Trägers zu stützen, und einen zweiten Teil (12a), der dazu ausgelegt ist, den M. quadriceps des Trägers zu stützen, wobei das zweite Flächengebilde (14) dazu ausgelegt ist, sich bei Gebrauch entlang eines wesentlichen Teils des iliotibialen Bands des Trägers zu befinden, und das dritte Flächengebilde (28) dazu ausgestaltet ist, sich bei Gebrauch über dem Knie zu befinden.

2. Kompressionskleidungsstück (10) nach Anspruch 1, wobei das dritte Flächengebilde (30) eine größere Elastizität aufweist als das zweite Flächengebilde (14), um eine Beugung des Knies zu gestatten.

3. Kompressionskleidungsstück (10) nach Anspruch 1 oder 2, das ferner ein Taillenband (64) umfasst, das aus einem Flächengebilde aus dehnbarem Material mit einem ausgewählten Dehn- und Rückstellvermögen gefertigt ist, das so ausgestaltet ist, dass das Taillenband keinen elastischen Einsatz im Rückenteil des Kleidungsstücks umfassen muss.

4. Kompressionskleidungsstück (10) nach Anspruch 3, das ferner einen elastischen Einsatz im Vorderteil des Kleidungsstücks umfasst.

5. Kompressionskleidungsstück (10) nach Anspruch 3 oder 4, das zum Zweck einer Förderung der Belüftung oder einer Unterstützung des Abführens von Körperwärme ferner einen oder mehrere Netzeinsätze umfasst.

6. Kompressionskleidungsstück (10) nach Anspruch 5, wobei der Netzeinsatz (62) sich unterhalb des Taillenbands (64) am oberen Rückenteil befindet.

7. Kompressionskleidungsstück (10) nach Anspruch 5 oder 6, wobei ein zweiter Netzeinsatz im Schrittbereich bereitgestellt ist.

## Revendications

1. Vêtement de compression (10) permettant d'habiller une partie de corps inférieure d'un porteur, le vêtement de compression comportant :
un premier panneau (12) en matériau extensible relié à un deuxième panneau (14) de matériau extensible par une première couture (16) et une deuxième couture (18) ;
un troisième panneau (30) relié au deuxième panneau (14) par une troisième couture (28) ;
le matériau extensible du deuxième panneau (14) présentant une caractéristique de récupération d'élasticité supérieure par rapport au matériau extensible du premier panneau (12) ;
**caractérisé en ce que**, en utilisation, le premier panneau (12) en matériau extensible est adapté pour soutenir un groupe de muscles plus grands et plus puissants que le deuxième panneau (14), le premier panneau étant divisé en deux parties, une première partie (12b) adaptée pour soutenir le grand fessier et le tendon du jarret du porteur, et une seconde partie (12a) adaptée pour soutenir le quadriceps du porteur, le deuxième panneau (14) étant adapté en utilisation pour être situé le long d'une partie substantielle du tractus ilio-tibial du porteur, et le troisième panneau (28) étant conçu en utilisation pour être situé au-dessus du genou.

2. Vêtement de compression (10) selon la revendication 1, dans lequel le troisième panneau (30) présente une élasticité supérieure à celle du deuxième panneau (14) de façon à permettre une flexion du genou.

3. Vêtement de compression (10) selon la revendication 1 ou 2, comprenant en outre une ceinture (64) fabriquée à partir d'un panneau de matériau extensible présentant des caractéristiques de récupération élastique choisies, conçue de sorte que la ceinture ne nécessite pas l'inclusion d'un empiècement élastique à l'arrière du vêtement.

4. Vêtement de compression (10) selon la revendication 3, comprenant en outre un empiècement élastique à l'avant du vêtement.

5. Vêtement de compression (10) selon la revendication 3 ou 4, comprenant en outre un ou plusieurs empiècements en maille, dans le but d'aider la ventilation ou de faciliter la dissipation de la chaleur du corps.

6. Vêtement de compression (10) selon la revendication 5, dans lequel l'empiècement en maille (62) est situé en dessous de la ceinture (64) à l'arrière en haut.

7. Vêtement de compression (10) selon la revendication 5 ou 6, dans lequel un second empiècement en maille est fourni dans l'entrejambe.
